# EUROPÄISCHE PATENTANMELDUNG

(11) **EP 3 391 917 A1**
(43) Veröffentlichungstag der Anmeldung: **24.10.2018**
(21) Anmeldenummer: 17167496.3
(22) Anmeldetag: 21.04.2017
(51) Int. Cl.: A61M 1/12, H02J 50/00, H01F 38/14, H02J 5/00, H02J 7/00

(54) **EINRICHTUNG ZUM STEUERN EINER IMPLANTIERBAREN HERZPUMPE UND HERZPUMPENEINRICHTUNG**

(71) Anmelder: Berlin Heart GmbH, 12247 Berlin (DE)
(72) Erfinder: Frischke, Dr. Michael, 15834 Rangsdorf (DE); Peters, Oliver, 14129 Berlin (DE)
(74) Vertreter: Pfenning, Meinig & Partner mbB

(57) **Zusammenfassung**

Die Anmeldung betrifft eine Einrichtung (9) zum Steuern einer implantierbaren Herzpumpe (1). Die Einrichtung (9) umfasst ein mit der Herzpumpe (1) über eine transkutane Leitung verbindbares extrakorporales Steuergerät (6) zur Versorgung der Herzpumpe (1) mit elektrischer Energie. Die Einrichtung (6) weist eine mit einer externen Energieversorgung (22) verbindbare Ladeschale (8) mit zumindest einer Sendespule (12) auf. Das Steuergerät (6) weist eine Empfangsspule (15) auf. Bei hinreichend geringem Abstand wirken die Sendespule (12) und die Empfangsspule (15) so zusammen, dass das Steuergerät (6) durch eine drahtlose, induktive Energieübertragung von der Sendespule (12) zu der Empfangsspule (15) mit Energie versorgbar ist.

## Beschreibung

Die vorliegende Anmeldung liegt auf dem Gebiet der Medizintechnik und betrifft eine Einrichtung zum Steuern einer implantierbaren Herzpumpe. Außerdem betrifft die vorliegende Anmeldung eine Herzpumpeneinrichtung mit der Herzpumpe.

Aus dem Stand der Technik sind implantierbare Herzpumpen bekannt. Diese Herzpumpen kommen zum Einsatz, wenn eine Herzfunktion eines Patienten unterstützt oder ersetzt werden muss. Gängige Systeme, die hierbei verwendet werden, sind sogenannte VAD (ventricular assist devices). Derartige Herzpumpen können beispielsweise als sog. LVAD (left ventricular assist device), RVAD (right ventricular assist device) oder BiVAD (bi-ventricular assist device) ausgeführt sein. Diese Systeme umfassen neben der Herzpumpe, die in dem Patienten implantiert ist, in der Regel ein außerhalb eines Körpers des Patienten angeordnetes und mit der Herzpumpe über eine perkutane Leitung verbundenes Steuergerät. Das Steuergerät kann beispielweise einen integrierten Akkumulator (wiederaufladbare Batterie) aufweisen oder mit einem Akkumulator verbindbar sein, so dass die implantierte Herzpumpe durch das Steuergerät mit elektrischer Energie versorgt werden kann. Es kann zudem vorgesehen sein, dass einen Betrieb oder eine Steuerung der Herzpumpe betreffende Daten zwischen dem Steuergerät und der implantierten Herzpumpe übertragen werden. Durch die Übertragung dieser Daten können beispielsweise Betriebsparameter der Herzpumpe festgelegt werden oder von implantierten Sensoren erfasste Messdaten sein. Ein System verwandter Art ist beispielsweise in der Druckschrift EP 3 090 767 A1 beschrieben.

Es ist eine Aufgabe der vorliegenden Anmeldung eine Einrichtung zum Steuern einer implantierbaren Herzpumpe vorzuschlagen, die eine verbesserte Energieversorgung der Herzpumpe gewährleistet. Insbesondere soll durch die vorgeschlagene Einrichtung ein ausfallsicheres und tragbares Steuergerät mit erhöhtem Trage- und Bedienkomfort ermöglicht werden.

Diese Aufgabe wird gelöst durch eine Einrichtung zum Steuern einer implantierbaren Herzpumpe mit den Merkmalen des Anspruchs 1. Vorteilhafte Weiterbildungen ergeben sich mit den Merkmalen der abhängigen Ansprüche und der Ausführungsbeispiele.

Die vorgeschlagene Einrichtung zum Steuern einer implantierbaren Herzpumpe umfasst ein mit der Herzpumpe über eine transkutane Leitung verbindbares extrakorporales Steuergerät zur Versorgung der Herzpumpe mit elektrischer Energie. Die Einrichtung weist eine mit einer externen Energieversorgung verbindbare Ladeschale mit zumindest einer Sendespule auf. Das Steuergerät weist eine Empfangsspule auf. Bei hinreichend geringem Abstand wirken die Sendespule und die Empfangsspule so zusammen, dass das Steuergerät durch eine drahtlose, induktive Energieübertragung von der Sendespule zu der Empfangsspule mit Energie versorgbar ist. Die Ladeschale kann, muss aber nicht notwendigerweise eine gewölbte Oberfläche aufweisen. Es kann vorgesehen sein, dass die Ladeschale eine im Wesentlichen flächige Form hat, was aber nicht notwendigerweise der Fall sein muss. Die Ladeschale und das Steuergerät können einen Dockingmechanismus ausbilden, in dem Sinne, dass die Ladeschale dem Steuergerät für die Energieübertragung zugeordnet ist.

Indem die Einrichtung über die induktive Energieübertragung mit Energie versorgt werden kann, sind weitere Anschlüsse bekannter Art für eine Verbindung des Steuergeräts mit der externen Energieversorgung zur Energieversorgung nicht mehr zwingend notwendig. Die induktive Energieübertragung hat im Vergleich zu herkömmlichen Steckverbindungen beispielsweise den Vorteil, dass das Steuergerät zumindest zu diesem Zweck keine Buchsen aufweisen muss. Derartige Buchsen sind in der Regel schwer zu reinigen. Besonders bei am Körper zu tragenden Steuergeräten für implantierbare Herzpumpen kann eine regelmäßige gründliche Reinigung sowie Desinfektion eines Gehäuses des Steuergeräts notwendig sein. Diese Reinigung beziehungsweise Desinfektion des Steuergerätes kann also dadurch deutlich vereinfacht und beschleunigt werden, dass das Steuergerät der vorgeschlagenen Einrichtung derart geschaffen werden kann, dass dieses keine Buchsen oder zumindest im Vergleich zu bekannten Steuergeräten eine reduzierte Anzahl von Buchsen aufweist. Zudem kann das bei Steckverbindungen auftretende Problem einer begrenzten Anzahl von Steckzyklen durch die vorgeschlagene Einrichtung vermieden werden.

Zudem erlaubt die vorgeschlagene Einrichtung ein besonders komfortables und schnelles Herstellen einer Verbindung zwischen dem Steuergerät und der externen Stromversorgung. Die Einrichtung kann in einigen Ausführungen beispielsweise eine Ausrichtungshilfe aufweisen. Durch die Ausrichtungshilfe können die Ladeschale und das Steuergerät bei einem Anlegen des Steuergeräts an die Ladeschale in zumindest einer vordefinierten Orientierung zueinander zu liegen kommen. Durch die Ausrichtungshilfe kann beispielsweise eine ungewünschte Verdrehung des Steuergeräts und der Ladeschale zueinander verhindert werden. Somit kann durch die Ausrichtungshilfe eine zuverlässige Verbindung zwischen dem Steuergerät und der externen Energieversorgung hergestellt werden, ohne dass eine hohe Präzision beim Anordnen der Ladeschale und des Steuergeräts zueinander notwendig ist, was insbesondere von Vorteil ist, wenn eine motorische Geschicklichkeit des Patienten eingeschränkt ist. Im Vergleich zu steckergebundenen Systemen ist ferner ein schnelles und weitgehend achtloses und doch zuverlässiges Versorgen des Steuergeräts mit elektrischer Energie von der externen Energieversorgung möglich. Über die Sende- und die Empfangsspule wird typischerweise höchstens eine Leistung von 40 Watt übertragen.

Es kann beispielsweise vorgesehen sein, dass die Ausrichtungshilfe dadurch gebildet wird, dass die Ladeschale und das Steuergerät zumindest in Bereichen komplementär zueinander geformte Oberflächen- oder Randkonturen aufweisen. Die Oberflächen- oder Randkonturen können derart geformt sein, dass die Ladeschale und das Steuergerät durch Formschluss in der vordefinierten Orientierung zueinander zu liegen kommen. Die Oberflächen des Steuergeräts und der Ladeschale können beispielsweise korrespondierende Vertiefungen, beispielsweise zumindest ein Sackloch oder eine Nut, beziehungsweise Erhöhungen, beispielsweise zumindest einen Zapfen, Stift, Keil, Riegel, Rippe oder Bolzen, aufweisen. Die Oberflächen des Steuergeräts und der Ladeschale können beispielsweise jeweils mehrere korrespondierende Vertiefungen beziehungsweise Erhöhungen aufweisen. Die Erhöhungen und Vertiefungen können sich in einigen Ausführungen aus einer gekrümmten Oberfläche herausheben. Durch den Formschluss kann in besonders einfacher Weise verhindert werden, dass das Steuergerät und die Ladeschale sich in ungewünschter Weise zueinander verdrehen oder seitlich verschieben. Die Oberflächen- bzw. Randkonturen greifen in der Regel ineinander, wenn die Ladeschale auf das Steuergerät aufgelegt ist. Die Ausrichtungshilfe kann zudem zur Führung der Ladeschale in die vordefinierte Position dienen, während die Ladeschale an das Steuergerät angelegt wird.

In typischen Ausführungen weisen die Sendespule und die Empfangsspule in der vordefinierten Orientierung einen minimalen Abstand zueinander auf. Dieser Abstand ist in der Regel größer als 0. Somit kann eine besonders effiziente drahtlose Energieübertragung gewährleistet werden, da eine Effizienz der drahtlosen Energieübertragung typischerweise durch eine Länge der Übertragungsstrecke, d.h. durch einen Abstand zwischen der Sende- und der Empfangsspule, bestimmt wird.

Es kann vorgesehen sein, dass die Einrichtung ein Haltemittel umfasst. Das Haltemittel kann mit dem Steuergerät und/oder mit der Ladeschale verbunden oder als Teil des Steuergeräts bzw. der Ladeschale ausgebildet sein. Zudem kann das Haltemittel eingerichtet sein, die Ladeschale lösbar an dem Steuergerät zu halten, insbesondere wenn die Ladeschale zur Energieversorgung des Steuergeräts an dem Steuergerät anliegt. Das Haltemittel kann beispielsweise eingerichtet sein, die Ladeschale kraft- und/oder formschlüssig an dem Steuergerät halten.

Es kann vorgesehen sein, dass die Ausrichtungshilfe eine Verschiebung der Ladeschale relativ zum Steuergerät im Wesentlichen in einer Richtung in einer Auflageebene oder eine Verdrehung der Ladeschale verhindert. Das Haltemittel kann eingerichtet sein, ein Ablösen der Ladeschale vom Steuergerät in einer Richtung quer zu der Auflageebene verhindert.

Es kann außerdem vorgesehen sein, dass das Haltemittel die Ladeschale zuglösbar an dem Steuergerät hält. In diesem Fall löst sich die Ladeschale durch ein Ziehen an der Ladeschale von selbst. Da die Ladeschale in typischen Ausführungen durch ein Kabel mit der externen Energieversorgung verbunden ist, besteht auch immer ein Risiko, dass beispielsweise durch eine unkontrollierte Bewegung des Patienten und/oder einen ungewollten Zug auf das Kabel ein Ruck auf die Ladeschale ausgeübt wird. Somit lässt sich durch das zuglösbare Haltemittel ein Verletzungsrisiko des Patienten und ein Risiko einer Beschädigung der Einrichtung beim Gebrauch merklich reduzieren.

In einigen Ausführungen umfasst die Ladeschale zumindest einen ersten Magneten, insbesondere einen Permanentmagneten bzw. einen Ferromagneten, und das Steuergerät zumindest einen zweiten Magneten, insbesondere einen Permanentmagneten bzw. einen Ferromagneten. Der zumindest eine erste Magnet und der zumindest eine zweite Magnet können eingerichtet sein einander anzuziehen, insbesondere wenn die Ladeschale zur Energieversorgung des Steuergeräts an dem Steuergerät anliegt. In einigen Ausführungen ist zumindest einer der Magneten ein Permanentmagnet. Der erste und der zweite Magnet können in einigen Ausführungen die Ausrichtungshilfe bilden. Somit kann es vorgesehen sein, dass die vordefinierte Orientierung durch ein Verdrehen und/oder Verschieben des Steuergeräts und der Ladeschale zueinander infolge einer Anziehung der Magneten erfolgt.

Es kann auch vorgesehen sein, dass das Haltemittel den zumindest einen ersten Magneten und den zumindest einen zweiten Magneten umfasst. Der zumindest eine erste Magnet und der zumindest eine zweite Magnet können hierbei eingerichtet sein, die Ladeschale an dem Steuergerät zu halten, insbesondere zuglösbar zu halten. Typischerweise wird die Ladeschale durch die Magneten gehalten, wenn die Ladeschale zur Energieversorgung des Steuergeräts an dem Steuergerät anliegt.

Es kann außerdem vorgesehen sein, dass der zumindest eine erste Magnet und der zumindest eine zweite Magnet derart angeordnet und ausgebildet sind, dass die Ladeschale und das Steuergerät beim Anlegen der Ladeschale an das Steuergerät durch eine magnetische Anziehung zwischen dem ersten Magneten und dem zweiten Magneten in einer vordefinierten Orientierung zueinander zu liegen kommen.

Der erste Magnet und der zweite Magnet können somit in einigen Ausführungen eine Doppelrolle spielen, nämlich sowohl als Ausrichtungshilfe, beispielsweise zum Verhindern einer seitlichen Verschiebung oder Verdrehung der Ladeschale, als auch als Haltemittel, beispielsweise zum Verhindern eines Ablösens der Ladeschale von dem Steuergerät, dienen. Es können in anderen Ausführungen jedoch auch unterschiedliche Paare von Magneten in unterschiedlicher Funktion, d.h. entweder als Ausrichtungshilfe oder als Haltemittel, vorgesehen sein. In anderen Ausführungen kann das Haltemittel mechanisch ausgebildet sein und beispielsweise durch einen schwenkbaren Haltebügel gebildet werden.

In einigen Ausführungen weist das Steuergerät eine außenliegende Fläche auf. Es kann außerdem vorgesehen sein, dass die Ladeschale einen überstehenden Rand mit einer innenliegenden Randfläche aufweist. Das Steuergerät und die Ladeschale können zudem derart geformt sein, dass die innenliegende Randfläche der Ladeschale die außenliegende Fläche des Steuergeräts zumindest teilweise umgreift, insbesondere wenn die Ladeschale zum Versorgen des Steuergeräts mit Energie an dem Steuergerät anliegt. In diesem Fall können die Ladeschale und das Steuergerät zum Ausrichten und zum Verhindern von Verschiebungen oder Verdrehungen der Ladeschale nach dem Prinzip einer in eine Ausnehmung bzw. Nut greifenden Erhöhung zusammenwirken. Auf diese Weise können beispielsweise die komplementär zueinander geformten Randkonturen der Ausrichtungshilfe ausgebildet sein.

In einigen Ausführungen ist die Sendespule zumindest teilweise in dem überstehenden Rand der Ladeschale angeordnet. Dadurch kann eine platzsparende Anordnung erreicht werden, bei der ein Spuleninneres der Sendespule in einem Bereich außerhalb eines Gehäuses der Ladeschale angeordnet ist, wobei das Spuleninnere der Sendespule für eine Aufnahme des Steuergeräts und insbesondere von dessen Empfangsspule zugänglich ist.

Das Steuergerät und die Ladeschale können auch derart geformt sein, dass eine innenliegende Randfläche des Steuergeräts eine außenliegende Randfläche der Ladeschale zumindest teilweise und insbesondere vollständig umgreift, insbesondere wenn die Ladeschale zum Versorgen des Steuergeräts mit Energie an dem Steuergerät anliegt. In diesem Fall kann eine Verbindung zwischen der Ladeschale und dem Steuergerät beispielsweise dadurch hergestellt werden, dass die Ladeschale zumindest bereichsweise in das Steuergerät eingesteckt wird.

Das Steuergerät kann eine mit der Empfangsspule verbundene und mit der Herzpumpe verbindbare Energieverwaltungseinheit aufweisen. Das Steuergerät kann zusätzlich oder alternativ einen integrierten Akkumulator aufweisen. Der integrierte Akkumulator dient in der Regel einer Versorgung der Herzpumpe zum Überbrücken eines vergleichsweise kurzen Zeitintervalls, in dem keine weiteren Energiequellen mit dem Steuergerät verbunden sind. Der integrierte Akkumulator kann mit der Energieverwaltungseinheit verbunden sein. In einigen Ausführungen sind mehrere wie oben oder unten beschriebene interne Akkumulatoren vorgesehen. Der interne Akkumulator beziehungsweise die internen Akkumulatoren sind in der Regel nicht ohne Weiteres aus dem Steuergerät entnehmbar, insbesondere nicht steckbar. Der interne Akkumulator beziehungsweise die internen Akkumulatoren sind typischerweise in einem Gehäuse des Steuergeräts angeordnet und/oder nicht lösbar mit weiteren Bestandteilen des Steuergeräts, insbesondere mit der Energieverwaltungseinheit, verbunden.

In einigen Ausführungen ist die Energieverwaltungseinheit eingerichtet, eine Energieverwaltung der Einrichtung derart zu steuern, dass die Herzpumpe durch die externe Energieversorgung versorgt und/oder dass der integrierte Akkumulator durch die externe Energieversorgung aufgeladen wird, insbesondere wenn die Ladeschale an dem Steuergerät anliegt. Das Steuergerät und/oder die Ladeschale weist typischerweise eine Detektionseinheit auf, die eingerichtet ist zu erkennen, ob die externe Energieversorgung zur Verfügung steht und/oder ob sich die Ladeschale bzw. das Steuergerät zur induktiven Energieversorgung in der Nähe befindet.

Es kann zudem vorgesehen sein, dass die Energieverwaltungseinheit eingerichtet ist, die Energieverwaltung der Einrichtung derart zu steuern, dass die Herzpumpe durch den integrierten Akkumulator mit Energie versorgt wird, wenn die externe Energieversorgung nicht verfügbar ist und insbesondere wenn die Ladeschale nicht an dem Steuergerät anliegt.

Es kann auch vorgesehen sein, dass das Steuergerät einen Steckplatz zum Aufstecken eines Steckakkumulators aufweist. Der Steckplatz kann elektrische Kontakte zum elektrischen Verbinden des Steckakkumulators mit der Energieverwaltungseinheit aufweisen. Der Steckakkumulator ist typischerweise starr mit dem Steckplatz verbindbar, beispielsweise durch Form- oder durch Kraftschluss. Ferner kann die Energieverwaltungseinheit eingerichtet sein, die Energieverwaltung der Einrichtung derart zu steuern, dass die Herzpumpe durch die externe Energieversorgung versorgt und/oder dass der Steckakkumulator durch die externe Energieversorgung aufgeladen wird, wenn der Steckakkumulator aufgesteckt ist und gegebenenfalls wenn die Ladeschale an dem Steuergerät anliegt.

Es kann auch vorgesehen sein, dass die Energieverwaltungseinheit eingerichtet ist, die Energieverwaltung der Einrichtung derart zu steuern, dass die Herzpumpe durch den Steckakkumulator versorgt und/oder dass der integrierte Akkumulator durch den Steckakkumulator aufgeladen wird, wenn der Steckakkumulator aufgesteckt ist und gegebenenfalls wenn die Ladeschale nicht an dem Steuergerät anliegt.

Es kann vorgesehen sein, dass die Energieverwaltungseinheit eingerichtet ist zu erkennen, dass kein Steckakkumulator aufgesteckt ist. In diesem Fall kann es vorgesehen sein, dass die Energieverwaltungseinheit eingerichtet ist, die Energieverwaltung der Einrichtung derart zu steuern, dass die Herzpumpe durch den integrierten Akkumulator mit Energie versorgt wird, wenn die externe Energieversorgung nicht verfügbar ist und insbesondere wenn die Ladeschale nicht an dem Steuergerät anliegt.

Die beschriebene Energieverwaltungseinheit kann auch Verwendung finden in einer Einrichtung zum Steuern einer implantierbaren Herzpumpe, ohne dass beispielsweise eine Ladeschale und/oder eine drahtlose induktive Energieübertragung vorgesehen ist. Außerdem kann die Energieverwaltungseinheit grundsätzlich in einem Steuergerät eingesetzt werden, das keine Empfangsspule aufweist. Beispielsweise kann die beschriebene Energieverwaltungseinheit auch wie beschrieben verwendet werden, wenn die externe Energieversorgung beispielsweise kabelgebunden mit dem Steuergerät verbindbar ist.

In einigen Ausführungen weist die Einrichtung Tragemittel auf. Das Tragemittel kann beispielsweise ein Gürtel, ein Tragegurt, eine Tragetasche oder eine Klebverbindung sein. Das Steuergerät ist typischerweise an dem Tragemittel befestigt. Somit kann das Steuergerät sicher am Körper des Patienten gehalten und ein Verrutschen des Steuergeräts verhindert werden. Dadurch kann eine Durchtrittsstelle für die transkutane Verbindung zwischen dem Steuergerät und der Herzpumpe entlastet werden. Das oben beschriebene Haltemittel kann auch als Teil des Tragemittels ausgebildet sein.

Die Anmeldung betrifft auch ein System umfassend eine Einrichtung zum Steuern einer implantierbaren Herzpumpe wie sie oben oder unten beschrieben ist und ferner umfassend ein Spiralkabel. Die Ladeschale kann zur Versorgung der Ladeschale mit elektrischer Energie mit dem Spiralkabel verbunden sein. Das Spiralkabel kann zudem mit der externen Energieversorgung verbindbar sein. Ein Spiralkabel erlaubt eine erhöhte Bewegungsfreiheit des Patienten und reduziert das Risiko eines unkontrollierten und unerwünschten Zugs an der Ladeschale.

Das System kann auch die externe Energieversorgung umfassen. In einigen Ausführungen wird die externe Energieversorgung durch einen herkömmlichen Stromnetzanschluss gebildet und umfasst beispielsweise eine herkömmliche Steckdose und gegebenenfalls ein an diese Steckdose angeschlossenes Netzteil. Die externe Energieversorgung kann aber auch einen Wagen umfassen. Zudem kann der Wagen einen Akkumulator umfassen. Der Akkumulator kann zur Versorgung der Ladeschale mit elektrischer Energie mit dieser verbunden oder verbindbar sein. In diesem Fall kann eine Bewegungsfreiheit des Patienten deutlich erhöht werden, während das Steuergerät durch die externe Energieversorgung mit elektrischer Energie versorgt wird.

In typischen Ausführungen werden zwischen dem Steuergerät und der Herzpumpe neben der elektrischen Energie auch Daten ausgetauscht. Es kann beispielsweise vorgesehen sein, dass die Herzpumpe Sensoren aufweist und/oder dass zusätzliche implantierbare Sensoren vorgesehen sind. In diesem Fall kann es vorgesehen sein, dass das Steuergerät zum Empfang von Messwerten, die durch die Sensoren erfasst werden, eingerichtet ist.

In typischen Ausführungen sind neben der elektrischen Energie auch Daten zwischen dem Steuergerät und der Ladeschale austauschbar, insbesondere induktiv über die Sende- und die Empfangsspule. Diese Daten können bidirektional austauschbar sein.

Ausführungsbeispiele werden nachfolgend anhand der Abbildungen beschrieben. Es zeigen
- Fig. 1: eine schematische Ansicht einer implantierten Herzpumpe und eines Steuergeräts,
- Fig. 2: eine schematische Ansicht einer Einrichtung zum Steuern der Herzpumpe und die Herzpumpe,
- Fign. 3(a) bis (d): schematische Ansichten zur Illustration einer Energieverwaltung der Einrichtung,
- Fign. 4(a) und (b): schematische Längsschnitte von Ausführungsbeispielen des Steuergeräts und einer Ladeschale,
- Fign. 5(a) bis (c): schematische Längsschnitte weiterer Ausführungsbeispiele des Steuergeräts und der Ladeschale,
- Fign. 6(a) bis (c): schematische Querschnitte weiterer Ausführungsbeispiele des Steuergeräts und der Ladeschale und
- Fig. 7: eine schematische Ansicht der Ladeschale und einer mit der Ladeschale verbundenen externen Energieversorgung.

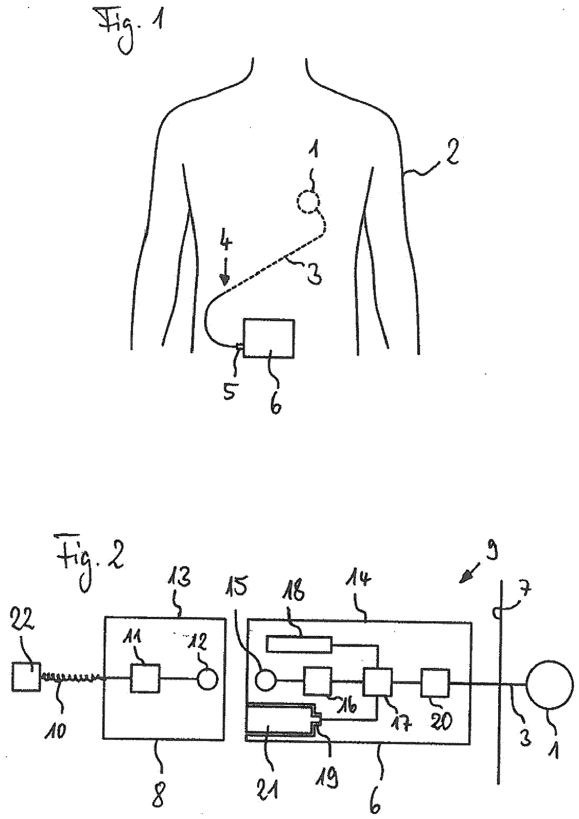

Figur 1 zeigt schematisch eine Ansicht einer Vorderseite eines Oberkörpers eines Patienten. Die Abbildung zeigt zudem eine Herzpumpe 1, die zur Unterstützung einer Herzfunktion des Patienten in dessen Körper 2 implantiert ist. Die Herzpumpe 1 weist einen in einem implantierbaren, biokompatiblen und fluiddicht verschweißten Pumpengehäuse angeordneten Motor und einen von dem Motor angetriebenen Rotor auf. Die Herzpumpe 1 kann beispielsweise als sogenanntes LVAD-System ausgeführt und derart im Körper 2 des Patienten angeordnet sein, dass durch eine Drehung des Rotors Blut vom linken Herzventrikel in die Aorta förderbar ist.

Die Herzpumpe 1 ist mit einer transkutanen Driveline 3, die teilweise unter einer Haut des Patienten verläuft, verbunden. Außerhalb einer Einstichstelle 4 ist die Driveline 3 über einen Stecker 5 mit einem Steuergerät 6 verbunden. Das Steuergerät 6 versorgt die Herzpumpe 1 über die Driveline 3 mit elektrischer Energie und mit Betriebsparametern, durch die beispielsweise eine von der Herzpumpe 1 in einem Zeitintervall von dem Herzventrikel in die Aorta zu fördernde Blutmenge festgelegt wird. In Ausführungen, in denen die Herzpumpe 1 oder weitere implantierte Geräte Sensoren aufweisen, können zudem von den Sensoren erfasste Informationen über die Driveline 3 an das Steuergerät 6 übertragen werden.

Das Steuergerät 6 ist in typischen Ausführungen außen am Körper 2 des Patienten tragbar ausgeführt. Zu diesem Zweck kann das Steuergerät 6 zum Beispiel mit einer Halterung verbunden sein und über diese Halterung mit dem Körper 2 des Patienten verbunden sein. Beispielsweise kann die Halterung ein Gürtel, ein Tragegurt, eine Tragetasche oder eine Klebverbindung sein, was in der Fig. 1 nicht dargestellt ist.

Figur 2 zeigt eine weitere schematische Ansicht der Herzpumpe 1, der Driveline 3 und des Steuergeräts 6. Wiederkehrende Merkmale sind in dieser und den nachfolgenden Abbildungen mit den gleichen Bezugszeichen versehen. Zudem ist schematisch eine Hautoberfläche 7 des Patienten dargestellt. Das Steuergerät 6 und eine Ladeschale 8 sind Bestandteile einer Einrichtung 9 zum Steuern der Herzpumpe 1. Die Einrichtung 9 und die Herzpumpe 1 bilden eine Herzpumpeneinrichtung.

Die Ladeschale 8 umfasst ein Sendemodul 11, das über ein Kabel 10 mit einer externen Energieversorgung 22 verbunden ist. Das Kabel 10 ist in dem gezeigten Ausführungsbeispiel als Spiralkabel ausgeführt. Weiterhin umfasst die Ladeschale 8 eine mit dem Sendemodul 11 kabelgebunden verbundene Sendespule 12. Das Sendemodul 11 und die Sendespule 12 sind in einem Gehäuse 13 der Ladeschale 8 angeordnet.

Das Steuergerät 6 weist ebenfalls ein Gehäuse 14 auf. In dem Gehäuse 14 sind eine Empfangsspule 15, ein mit der Empfangsspule 15 kabelgebunden verbundenes Empfangsmodul 16, eine mit dem Empfangsmodul 16 kabelgebunden verbundene Energieverwaltungseinheit 17, einen mit der Energieverwaltungseinheit 17 kabelgebunden verbundenen und in das Steuergerät 6 integrierten Akkumulator 18, einen mit der Energieverwaltungseinheit 17 kabelgebunden verbundenen Steckplatz 19 sowie eine mit der Energieverwaltungseinheit 17 kabelgebunden verbundene Kontrolleinheit 20. Über die Kontrolleinheit 20 ist das Steuergerät 6 kabelgebunden mit der Driveline 3 und der Herzpumpe 1 verbunden. Das Steuergerät 6 weist außerdem zumindest eine nicht dargestellte Speichereinrichtung auf, um beispielsweise von den implantierten Sensoren erfasste Messwerte und anderweitige Daten zwischenzuspeichern. Ein Steckakkumulator 21 ist zudem in das Steuergerät 6 eingesteckt und mit dem Steckplatz 19 mechanisch und über entsprechend vorgesehene Kontakte elektrisch verbunden.

Das Steuergerät 6 kann durch die Ladeschale 8 mit elektrischer Energie versorgt werden, so dass die Herzpumpe 1 mit Energie versorgt werden kann oder so dass Operationen zum Betrieb der Herzpumpe 1 oder zum Speichern oder Auslesen von Informationen durchgeführt werden können. Zu diesem Zweck wirken die Sendespule 12 und die Empfangsspule 15 derart zusammen, dass Energie zwischen von der Sendespule 12 zu der Empfangsspule 15 übertragbar ist. Um eine durch den Abstand zwischen der Sendespule 12 und der Empfangsspule 15 bestimmte Übertragungsstrecke kurz zu halten, sind die Sendespule 12 und die Empfangsspule 15 möglichst nah unterhalb des jeweiligen Gehäuses 13, 14 angeordnet. Es kann in anderen Ausführungen auch vorgesehen sein, dass die Sendespule 12 und/oder die Empfangsspule 15 als Bestandteil des zugehörigen Gehäuses 13, 14 ausgebildet ist. Zum Beispiel können Spulen durch in ein Kunststoffgehäuseteil eingelassene Leiterbahnen gebildet werden. Elektrische Energie wird in der Regel übertragen, während die Ladeschale 8 und das Steuergerät 6 nah zueinander angeordnet sind. Hierbei liegen typischerweise aber nicht notwendigerweise die Gehäuse 13, 14 der Ladeschale und des Steuergeräts 6 berührend aneinander an.

Das Sendemodul 11 und das Empfangsmodul 16 umfassen typischerweise einen Mikroprozessor und regeln während einer induktiven Energieübertragung zwischen den Spulen 12, 15 die übertragene elektrische Leistung. Zudem kann das Sendemodul 11 und/oder das Empfangsmodul 16 einen Detektionsschaltkreis aufweisen, durch den erkannt wird, dass sich das Steuergerät 6 beziehungsweise die Ladeschale 8 in der Nähe befindet. Es kann vorgesehen sein, dass die Ladeschale 8 oder das Steuergerät 6 ein optisches oder akustisches Signal abgibt, wenn eine Nähe des Steuergeräts 6 beziehungsweise der Ladeschale 8 erkannt wird oder wenn diese Nähe nicht mehr erkannt wird.

Es kann auch vorgesehen sein, dass über die Sendespule 12 und die Empfangsspule 15 Daten induktiv zwischen der Ladeschale 8 und dem Steuergerät 6 übertragen werden. Beispielsweise kann es vorgesehen sein, dass zum Programmieren des Steuergeräts 6, insbesondere zum Anpassen von Betriebsparametern der Herzpumpe 1 oder Regelungsvorschriften zur Steuerung der Betriebsparameter der Herzpumpe 1, Daten über die induktive Übertragungsstrecke von der Sendespule 12 zur Empfangsspule 15 übertragen werden. Es kann aber auch vorgesehen sein, dass Daten in umgekehrter Richtung, d.h. von der Empfangsspule 15 zur Sendespule 12, übertragen werden. Dieser Übertragungsweg kann beispielsweise zum Auslesen von Sensordaten, die von den implantierten Sensoren erfasst wurden, genutzt werden. Diese Datenübertragung zwischen dem Steuergerät 6 und der Ladeschale 8 wird in der Regel durch das Sendemodul 11 und das Empfangsmodul 16 oder über die Energieverwaltungseinheit 17 gesteuert.

Zusätzlich kann auch eine Datenübertragung zwischen dem Steuergerät 6 und der Ladeschale 8 mittels anderen drahtlosen Übertragungsarten, insbesondere Bluetooth oder ZigBee, oder auch kabelgebundenen Übertragungsarten vorgesehen sein, die in den Abbildungen nicht dargestellt sind. In diesem Fall weisen das Steuergerät 6 und die Ladeschale 8 entsprechend geeignete Module auf. Diese Datenübertragung zwischen dem Steuergerät 6 und der Ladeschale 8 wird in der Regel durch das Sendemodul 11 und das Empfangsmodul 16 oder über die Energieverwaltungseinheit 17 gesteuert.

Außerdem umfasst die Ladeschale zum Senden und/oder Empfangen der oben beschriebenen Daten zumindest eine entsprechend geeignete Schnittstelle zu einem weiteren Gerät, beispielsweise einem klinischen PC oder einem Tablet, wobei diese Schnittstelle in der Abbildung nicht dargestellt ist und beispielsweise als herkömmliche Steckverbindung oder als drahtlose Schnittstelle ausgebildet sein kann. Typischerweise können über die Schnittstelle Daten mit einer Übertragungsrate von mindestens 1 kByte/s übertragen werden, insbesondere bidirektional.

In einigen Ausführungen wird die Herzpumpe 1 in einem normalen Betriebszustand durch den Steckakkumulator 21 mit Energie versorgt. Der Steckakkumulator 21 kann die Herzpumpe für eine gewisse Zeit mit Energie versorgen, muss allerdings regelmäßig aufgeladen und/oder gegen einen geladenen Steckakkumulator ausgetauscht werden. Eine in dem im Steuergerät 6 integrierten Akkumulator 18 speicherbare elektrische Energiemenge ist in typischen Ausführungen hingegen nicht ausreichend, um die Herzpumpe 1 langfristig mit Energie zur versorgen. In typischen Ausführungen ist die Herzpumpe 1 allein über den integrierten Akkumulator 18 lediglich kurzfristig, beispielsweise für höchstens 15 Minuten, mit Energie versorgbar, wenn der integrierte Akkumulator 18 zu Beginn vollständig geladen war. Somit kann durch den integrierten Akkumulator 18 ein Betrieb und eine Energieversorgung der Herzpumpe 1 gewährleistet werden, wenn keine anderweitigen Energiequellen zum Betrieb der Herzpumpe 1 verfügbar sind. Dadurch kann beispielsweise ein Zeitraum überbrückt werden, in dem der Steckakkumulator 21 gewechselt werden muss.

Die Kontrolleinheit 20 umfasst typischerweise zumindest einen Mikrocontroller. In der Regel werden durch die Kontrolleinheit 20 Betriebsparameter der Herzpumpe 1 gesteuert und errechnet, beispielsweise anhand von durch die Sensoren erfassten und über die Driveline 3 an das Steuergerät 6 übertragenen Messwerten.

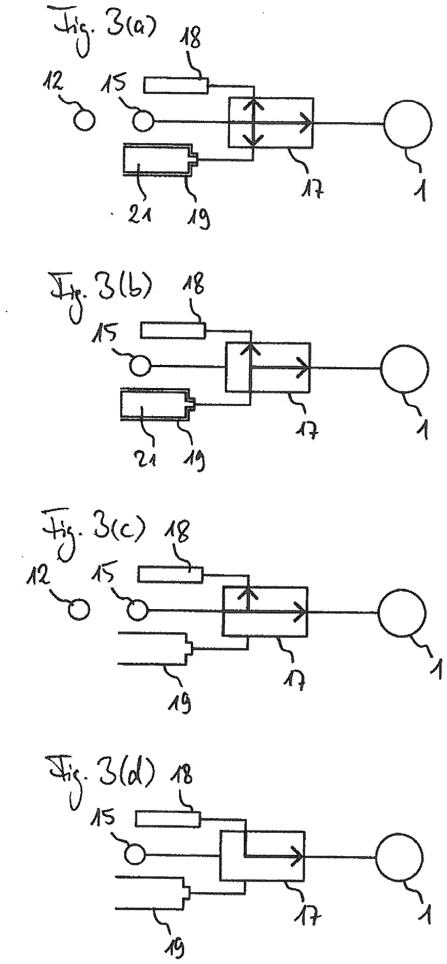

Die Energieverwaltungseinheit 17 kann beispielsweise zumindest einen Mikrocontroller aufweisen. Die Energieverwaltungseinheit 17 ist eingerichtet, eine Energieverwaltung der Einrichtung 9 zu steuern. Insbesondere kann durch die Energieverwaltungseinheit 17 eine Energieversorgung der Herzpumpe 1 abhängig von in gewissen Betriebssituationen verfügbaren Energiequellen zu regeln. Diese Betriebssituation und die von der Energieverwaltungseinheit 17 festgelegte Energieverwaltung der Einrichtung 9 ist in den Fign. 3(a) bis (d) schematisch illustriert.

Figur 3(a) illustriert eine erste Betriebssituation, in der die Ladeschale 8 auf das Steuergerät 6 aufgelegt ist. In dieser Situation befindet sich die Sendespule 12 in der Nähe der Empfangsspule 15, was durch das Sendemodul 11 und/oder das Empfangsmodul 16 detektiert wird. Zudem ist in dieser Betriebssituation der Steckakkumulator 21 weiterhin in den Steckplatz 19 eingesteckt, was beispielsweise durch die Energieverwaltungseinheit 17 erkannt wird. Die Energieverwaltungseinheit 17 steuert hierbei die Energieverwaltung so, dass sowohl der integrierte Akkumulator 18 als auch der Steckakkumulator 21 durch die induktive Energieübertragungsstrecke, d.h. über die Sende- und Empfangsspulen 12, 15, aufgeladen werden. Zudem wird in diesem Fall die Herzpumpe 1 über die induktive Energieübertragungsstrecke mit Energie versorgt.

Figur 3(b) illustriert eine zweite Betriebssituation. In diesem Fall ist die Ladeschale 8 nicht auf das Steuergerät 6 aufgelegt, was durch das Sendemodul 11 und/oder das Empfangsmodul 16 detektiert wird. Allerdings ist in dieser Betriebssituation der Steckakkumulator 21 in den Steckplatz 19 eingesteckt. Die Energieverwaltungseinheit 17 steuert hierbei die Energieverwaltung so, dass der integrierte Akkumulator 18 durch eine Übertragung elektrischer Energie von dem Steckakkumulator 21 aufgeladen wird. Zudem wird in diesem Fall die Herzpumpe 1 durch den Steckakkumulator 21 mit Energie versorgt.

Figur 3(c) illustriert eine dritte Betriebssituation, in der die Ladeschale 8 auf das Steuergerät 6 aufgelegt ist. In dieser Betriebssituation ist der Steckakkumulator 21 nicht in den Steckplatz 19 eingesteckt. Die Energieverwaltungseinheit 17 steuert in diesem Fall die Energieverwaltung so, dass der integrierte Akkumulator 18 durch die induktive Energieübertragungsstrecke aufgeladen wird. Zudem wird in diesem Fall die Herzpumpe 1 über die induktive Energieübertragungsstrecke mit Energie versorgt.

Figur 3(d) illustriert eine vierte Betriebssituation. In diesem Fall ist die Ladeschale 8 nicht auf das Steuergerät 6 aufgelegt. Zudem ist in dieser Betriebssituation der Steckakkumulator 21 nicht in den Steckplatz 19 eingesteckt. Die Energieverwaltungseinheit 17 steuert die Energieverwaltung nun so, dass die Herzpumpe 1 durch den integrierten Akkumulator 18 mit Energie versorgt wird.

Die Energieverwaltungseinheit kann auch in der beschriebenen Weise eingesetzt werden, wenn das Steuergerät 6 statt der induktiven Ladestrecke und der Sende- bzw. Empfangsspule über eine kabelgebundene Verbindung mit der externen Energieversorgung verbindbar ist, beispielsweise über eine herkömmliche Steckverbindung.

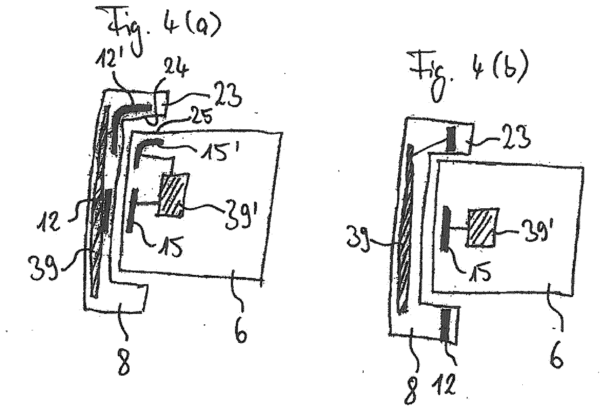

In Fign. 4(a) und (b) sind weitere schematische Ansichten des Steuergeräts 6 und der Ladeschale 8 im Querschnitt gezeigt. In den gezeigten Darstellungen liegt die Ladeschale 8 weitgehend an dem Steuergerät 6 an. In diesen Ausführungen weist die Ladeschale 8 einen Rand 23 auf. Der Rand 23 bildet an seiner Innenseite eine innenliegende Randfläche 24 und ist derart ausgebildet, dass die Randfläche 24 eine außenliegende Fläche 25 des Steuergeräts 6 umgreift. Die Randkonturen des Steuergeräts 6 und der Ladeschale 8 werden durch die Fläche 25 bzw. die Randfläche 24 gebildet. Diese Randkonturen sind in ihrer Form komplementär ausgebildet, so dass die Randkonturen eine Ausrichtungshilfe bilden, indem diese eine relative Orientierung der Ladeschale 8 und des Steuergeräts 6 festlegen. Durch die Randkonturen wird ein Verdrehen und ein Verschieben der Ladeschale 8 verhindert, wenn die Ladeschale 8 an dem Steuergerät 6 anliegt. Die Ladeschale 8 und das Steuergerät 6 greifen hierbei formschlüssig ineinander.

Durch die Ausrichtungshilfe kommen die Ladeschale 8 und das Steuergerät 6 in einer vordefinierten Orientierung und Position zu liegen. Die vordefinierte Orientierung und Position ist so gewählt, dass die Empfangsspule 15 und die Sendespule 12 einen minimalen Abstand zueinander aufweisen. In typischen Ausführungen beträgt dieser Abstand 30 mm oder weniger. In dem in Fig. 4(a) dargestellten Beispiel weist die Ladeschale 8 die Sendespule 12 und eine zweite Sendespule 12' auf. Die zweite Sendespule 12' ist gekrümmt und ist teilweise in dem Rand 25 der Ladeschale 8 angeordnet. Die erste und die zweite Sendspule 12, 12' sind zur induktiven Energie- und Datenübertragung jeweils einer von zwei Empfangsspulen 15, 15' des Steuergeräts 6 zugeordnet. Schematisch sind zudem den Spulen zugeordnete Ladeschaltkreise gezeigt, die mit den Bezugszeichen 39 und 39'versehen sind. Der Ladeschaltkreis 39 der Ladeschale 8 kann auch ein Netzteil umfassen.

In der Ausführung der Fig. 4(b) weist die Ladeschale 8 lediglich eine Sendespule 12 auf, die umlaufend in dem Rand 25 der Ladeschale 8 angeordnet ist. Das Steuergerät 6 weist eine zugehörige Empfangsspule 15 auf, die in ein Spuleninneres der Sendespule 12 hineinragt, wenn die Ladeschale 8 auf das Steuergerät 6 aufgelegt ist. Die Sende- und die Empfangsspule 12, 15 liegen hierbei in einer gemeinsamen Ebene.

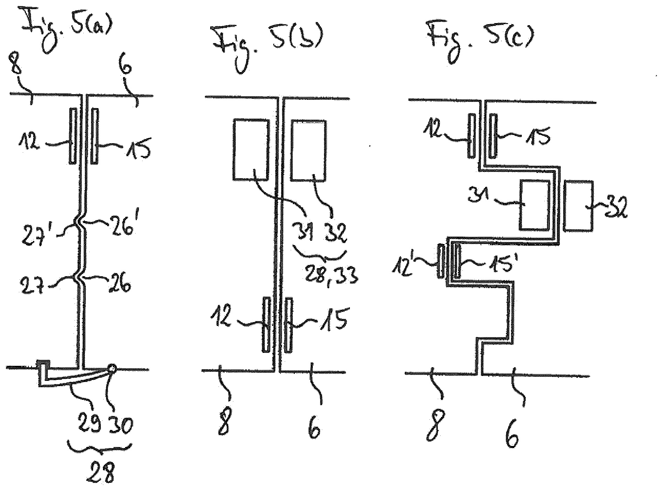

Weitere Ausführungsbeispiele ergeben sich mit den schematischen Darstellungen der Fign. 5(a) bis (c). Fig. 5(a) zeigt einen Querschnitt durch einen Auflagebereich des Steuergeräts 6 und der Ladeschale 8. Eine Oberfläche des Steuergeräts 6 weist Erhöhungen 26, 26', die mit Vertiefungen 27, 27' auf einer Oberfläche der Ladeschale 8 korrespondieren und die in die Vertiefungen 27, 27 eingreifen. Durch die korrespondierenden Oberflächen wird eine seitliches Verschieben und Verdrehen der Ladeschale 8 relativ zu dem Steuergerät 6 verhindert. Auf diese Weise bilden die Erhöhungen 26, 26' und die Vertiefungen 27, 27' eine Ausrichtungshilfe. Zudem ist ein mit dem Steuergerät 6 verbundenes Haltemittel 28 gezeigt, das einen schwenkbaren Haken 29 und ein Scharnier 30 umfasst. Durch das Haltemittel 28 wird die Ladeschale 8 in Position gehalten, wenn die Ladeschale 8 auf das Steuergerät 6 aufgelegt ist. In dieser Position kommen die Empfangsspule 15 und die Sendespule 12 in einem minimalen Abstand zueinander zu liegen.

In dem Ausführungsbeispiel gemäß Fig. 5(b) wird das Haltemittel 28 dadurch gebildet, dass die Ladeschale 8 einen ersten Magneten 31 und das Steuergerät 6 einen zweiten Magneten 32 aufweist. Das Haltemittel 28 ist durch Ziehen an der Ladeschale 8 lösbar. Beispielsweise können beide Magneten 31, 32 Permanentmagneten sein, wobei die Magneten 31, 32 einander in der gezeigten Anordnung anziehen. Somit wird die Ladeschale 8 durch die Magneten 31, 32 an dem Steuergerät 6 gehalten. Zudem wird durch die Anziehung der Magneten 31, 32 eine relative Orientierung der Ladeschale 8 zum Steuergerät 6 festgelegt, so dass die Magneten 31, 32 auch eine Ausrichtungshilfe 33 darstellen. Die Oberflächen der Ladeschale 8 und des Steuergeräts 6 sind in der Darstellung zwar glatt, können aber in oben oder unten beschriebener Weise konturiert sein.

In der Ausführung, die in Fig. 5(c) dargestellt ist, ist die Ladeschale 8 als Stecker mit stiftartigen Erhöhungen ausgebildet. Die Erhöhungen kommen in korrespondierenden Vertiefungen des Steuergeräts 6 zu liegen, wenn die Ladeschale 8 in das Steuergerät 6 eingesteckt ist. Die Erhöhungen und Vertiefungen verhindern ein seitliches Verschieben oder Verdrehen der Ladeschale 8 und bilden somit eine Ausrichtungshilfe und führen die Ladeschale 8 beim Einstecken in die vordefinierte Position. In dieser Anordnung kommen Sendespule 12, 12' und Empfangsspulen 15, 15' paarweise zueinander benachbart zu liegen. Zusätzlich sind Magneten 31, 32 vorgesehen, die ein Haltemittel oben beschriebener Art bilden.

In dem in Fig. 5(c) gezeigten Beispiel ist die Sendespule 12' versenkt, d.h. unterhalb einer Vertiefung einer Oberfläche der Ladeschale 8 angeordnet. Indem die Sendespule 12' vertieft angeordnet ist, ist diese nach der Energieübertragung und wenn die Ladeschale 8 nicht mehr in das Steuergerät 6 eingesteckt ist, für einen Anwender nicht ohne weiteres berührbar. Da es vorkommen kann, dass sich die Oberfläche der Ladeschale 8 im Bereich der Sendespule 12' während der Energieübertragung merklich erwärmt, wird durch die versenkte Anordnung der Sendespule 12' eine Sicherheit des Anwenders erhöht, indem verhindert wird, dass der Anwender nach der induktiven Energieübertragung versehentlich gegen den erwärmten Bereich der Oberfläche der Ladeschale 8 stößt. Dasselbe kann in weiteren Ausführungen sinngemäß für weitere Sendespulen und eine oder mehr Empfangsspule vorgesehen sein.

Es kann somit in einigen Ausführungen vorgesehen sein, dass die Sende- und/oder die Empfangsspule versenkt, d.h. unterhalb eines vertieften Bereichs einer Oberfläche der Ladeschale bzw. des Steuergeräts angeordnet sind. In einigen Ausführungen sind sohl die Sende- als auch die Empfangsspule versenkt angeordnet. So kann ein die Sende- beziehungsweise die Empfangsspule abdeckender Bereich der Oberfläche nicht berührbar sein. Es kann in einigen Ausführungen vorgesehen sein, dass die übertragene Leistung und/oder die Wärmeverluste derart gering sind, dass keine kritische Erwärmung der Oberfläche der Ladeschale und des Steuergeräts auftritt. Zusätzlich oder alternativ kann auch ein Kühlungskonzept vorgesehen sein, durch das eine kritische Aufwärmung der Oberflächen verhindert wird.

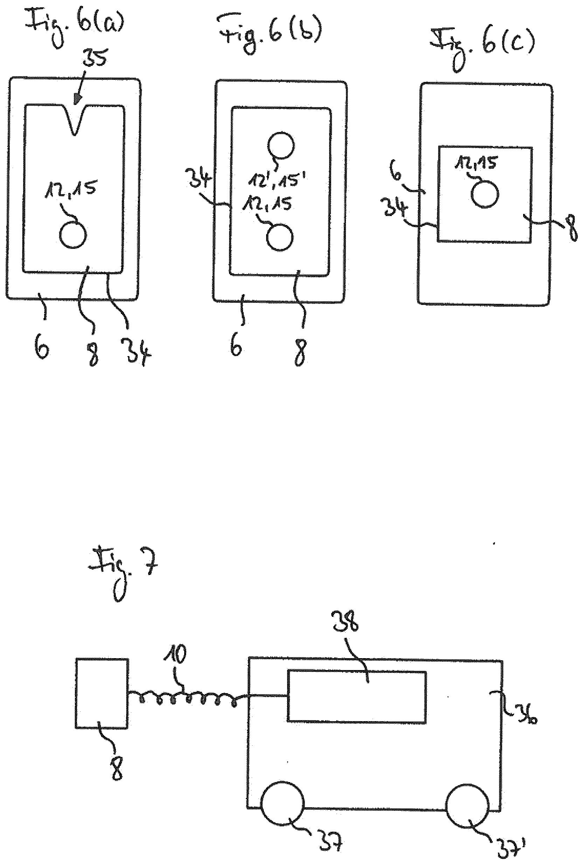

Weitere Ausführungen zeigen Fign. 6(a) bis (c). In diesen Ausführungen wird die Ausrichtungshilfe durch korrespondierende Randkonturen 34 des Steuergeräts 6 und der Ladeschale 8 gebildet. Die Randkonturen 34 bilden in dem in Fig. 6(a) gezeigte Ausführungsbeispiel einen Bereich 35 aus, durch den eine Symmetrie gebrochen wird. Somit kann die Ladeschale 8 in nur einer möglichen vordefinierten Orientierung auf dem Steuergerät 6 zu liegen kommen. Diese Orientierung ist derart gewählt, dass die Empfangsspule 15 und Sendespule 12 übereinander zu liegen kommen.

In dem in Fig. 6(b) gezeigten Ausführungsbeispiel sind aufgrund der Symmetrie der Randkonturen 34 zwei verschiedene vordefinierte Orientierungen möglich. Die Ladeschale 8 weist zwei Sendespulen 12, 12' und das Steuergerät 6 weist zwei Empfangsspulen 15, 15' auf. Die Spulen sind so angeordnet, dass sich beide Orientierungen zum Versorgen des Steuergeräts 6 mit elektrischer Energie eignen, da in jeder der Orientierungen Spulen paarweise übereinander zu liegen kommen. Ein weiteres Beispiel zeigt Fig. 6(c). Hierbei sind die Randkonturen 34 annähernd quadratisch, so dass die Ladeschale 8 in einer von vier möglichen Orientierungen auf das Steuergerät 6 aufgelegt werden kann. In diesem Fall sind die Sendespule 12 und die Empfangsspule 15 in Bezug auf die Randkontur 34 jeweils mittig angeordnet, so dass diese Spulen in allen vier Orientierungen übereinander zu liegen kommen.

Die externe Energieversorgung kann beispielsweise als herkömmliche Steckdose oder auch als Wagen 36 ausgebildet sein. Figur 7 zeigt eine Anordnung, in der die externe Energieversorgung als Wagen 36 ausgebildet ist, wobei der Wagen 36 Rollen 37, 37' und einen Akkumulator 38 aufweist. Der Akkumulator 38 kann eine größere Menge elektrischer Energie speichern als der Steckakkumulator 21 und der integrierte Akkumulator 18 im vollständig geladenen Zustand. Somit kann der Akkumulator 38 das Steuergerät 6 und die Herzpumpe 1 über einen vergleichsweise langen Zeitraum mit Energie versorgen, ohne dass der Akkumulator 38 ausgetauscht oder aufgeladen werden muss. Der Akkumulator 38 ist wie gezeigt über das Spiralkabel 10 mit der Ladeschale 8 zur Versorgung der Ladeschale 8, des Steuergeräts 6 und der Herzpumpe 1 mit elektrischer Energie verbunden.

Lediglich in den Ausführungsbeispielen offenbarte Merkmale der verschiedenen Ausführungsformen können miteinander kombiniert und einzeln beansprucht werden.

## Patentansprüche

1. Einrichtung (9) zum Steuern einer implantierbaren Herzpumpe (1), umfassend ein mit der Herzpumpe (1) über eine transkutane Leitung verbindbares extrakorporales Steuergerät (6) zur Versorgung der Herzpumpe (1) mit elektrischer Energie, wobei die Einrichtung (9) eine mit einer externen Energieversorgung (22) verbindbare Ladeschale (8) mit zumindest einer Sendespule (12) aufweist, wobei das Steuergerät (6) eine Empfangsspule (15) aufweist, wobei die Sendespule (12) und die Empfangsspule (15) bei hinreichend geringem Abstand derart zusammenwirken, dass das Steuergerät (6) durch eine drahtlose, induktive Energieübertragung von der Sendespule (12) zu der Empfangsspule (15) mit Energie versorgbar ist.

2. Einrichtung (9) nach Anspruch 1, **gekennzeichnet durch** eine Ausrichtungshilfe, durch die die Ladeschale (8) und das Steuergerät (6) bei einem Anlegen des Steuergeräts (6) an die Ladeschale (8) in zumindest einer vordefinierten Orientierung zueinander zu liegen kommen.

3. Einrichtung (9) nach Anspruch 2, **dadurch gekennzeichnet, dass** die Ausrichtungshilfe dadurch gebildet wird, dass die Ladeschale (8) und das Steuergerät (6) zumindest in Bereichen derart komplementär zueinander geformte Oberflächen- oder Randkonturen aufweisen, dass die Ladeschale (8) und das Steuergerät (6) durch Formschluss in der vordefinierten Orientierung zueinander zu liegen kommen.

4. Einrichtung (9) nach einem der Ansprüche 1 bis 3, **dadurch gekennzeichnet, dass** die Ladeschale (8) zumindest einen ersten Magneten (31), insbesondere einen Permanentmagneten bzw. einen Ferromagneten, und das Steuergerät (6) zumindest einen zweiten Magneten (32), insbesondere einen Permanentmagneten bzw. einen Ferromagneten, aufweist, wobei der zumindest eine erste Magnet (31) und der zumindest eine zweite Magnet (32) eingerichtet sind einander anzuziehen, wenn die Ladeschale (8) zur Energieversorgung des Steuergeräts (6) an dem Steuergerät (6) anliegt.

5. Einrichtung (9) nach Anspruch 4, **dadurch gekennzeichnet, dass** der zumindest eine erste Magnet (31) und der zumindest eine zweite Magnet (32) derart angeordnet und ausgebildet sind, dass die Ladeschale (8) und das Steuergerät (6) beim Anlegen der Ladeschale (8) an das Steuergerät (6) durch eine magnetische Anziehung zwischen dem ersten Magneten (31) und dem zweiten Magneten (32) in einer vordefinierten Orientierung zueinander zu liegen kommen.

6. Einrichtung (9) nach einem der vorangehenden Ansprüche, **dadurch gekennzeichnet, dass** die Sendespule (12) und die Empfangsspule (15) in der vordefinierten Orientierung einen minimalen Abstand zueinander aufweisen.

7. Einrichtung (9) nach einem der Ansprüche 1 bis 6, **dadurch gekennzeichnet, dass** das Steuergerät (6) eine außenliegende Fläche (25) aufweist und dass die Ladeschale (8) einen überstehenden Rand (23) mit einer innenliegenden Randfläche (24) aufweist, wobei das Steuergerät (6) und die Ladeschale (8) derart geformt sind, dass die innenliegende Randfläche (24) der Ladeschale (8) die außenliegende Fläche (25) des Steuergeräts (6) zumindest teilweise umgreift, wenn die Ladeschale (8) zum Versorgen des Steuergeräts (6) mit Energie an dem Steuergerät (6) anliegt.

8. Einrichtung (9) nach Anspruch 7, **dadurch gekennzeichnet, dass** die Sendespule (12) zumindest teilweise in dem überstehenden Rand (23) der Ladeschale (8) angeordnet ist.

9. Einrichtung (9) nach einem der Ansprüche 1 bis 8, **gekennzeichnet durch** ein Haltemittel (28), das mit dem Steuergerät (6) und/oder mit der Ladeschale (8) verbunden oder als Teil des Steuergeräts (6) bzw. der Ladeschale (8) ausgebildet ist, wobei das Haltemittel (28) eingerichtet ist, die Ladeschale (8) lösbar an dem Steuergerät (6) zu halten, wenn die Ladeschale (8) zur Energieversorgung des Steuergeräts (6) an dem Steuergerät (6) anliegt.

10. Einrichtung (9) nach Anspruch 9, sofern dieser auf Anspruch 4 rückbezogen ist, **dadurch gekennzeichnet, dass** das Haltemittel (28) den zumindest einen ersten Magneten (31) und den zumindest einen zweiten Magneten (32) umfasst, wobei der zumindest eine erste Magnet (31) und der zumindest eine zweite Magnet (32) eingerichtet sind, die Ladeschale (8) zuglösbar an dem Steuergerät (6) zu halten, wenn die Ladeschale (8) zur Energieversorgung des Steuergeräts (6) an dem Steuergerät (6) anliegt.

11. Einrichtung (9) nach einem der Ansprüche 1 bis 10, **dadurch gekennzeichnet, dass** das Steuergerät (6) eine mit der Empfangsspule (15) verbundene und mit der Herzpumpe (1) verbindbare Energieverwaltungseinheit (17) und einen integrierten Akkumulator (18) aufweist, der mit der Energieverwaltungseinheit (17) verbunden ist, wobei die Energieverwaltungseinheit (17) eingerichtet ist, eine Energieverwaltung der Einrichtung (9) derart zu steuern, dass die Herzpumpe (1) durch die externe Energieversorgung (22) versorgt und/oder dass der integrierte Akkumulator (18) durch die externe Energieversorgung (22) aufgeladen wird, wenn die Ladeschale (8) an dem Steuergerät (6) anliegt.

12. Einrichtung (9) nach Anspruch 11, **dadurch gekennzeichnet, dass** die Energieverwaltungseinheit (17) eingerichtet ist, die Energieverwaltung der Einrichtung (9) derart zu steuern, dass die Herzpumpe (1) durch den integrierten Akkumulator (18) mit Energie versorgt wird, wenn die Ladeschale (8) nicht an dem Steuergerät (6) anliegt.

13. Einrichtung (9) nach einem der Ansprüche 11 oder 12, **dadurch gekennzeichnet, dass** das Steuergerät (6) einen Steckplatz (19) zum Aufstecken eines Steckakkumulators (21) aufweist, wobei der Steckplatz (19) elektrische Kontakte zum elektrischen Verbinden des Steckakkumulators (21) mit der Energieverwaltungseinheit (17) aufweist, wobei die Energieverwaltungseinheit (17) eingerichtet ist, die Energieverwaltung der Einrichtung (9) derart zu steuern, dass die Herzpumpe (1) durch die externe Energieversorgung (22) versorgt und/oder dass der Steckakkumulator (21) durch die externe Energieversorgung (22) aufgeladen wird, wenn der Steckakkumulator (21) aufgesteckt ist und wenn die Ladeschale (8) an dem Steuergerät (6) anliegt.

14. Einrichtung (9) nach Anspruch 13, **dadurch gekennzeichnet, dass** die Energieverwaltungseinheit (17) eingerichtet ist, die Energieverwaltung der Einrichtung (9) derart zu steuern, dass die Herzpumpe (1) durch den Steckakkumulator (21) versorgt und/oder dass der integrierte Akkumulator (18) durch den Steckakkumulator (21) aufgeladen wird, wenn der Steckakkumulator (21) aufgesteckt ist und wenn die Ladeschale (8) nicht an dem Steuergerät (6) anliegt.

15. Einrichtung (9) nach einem der Ansprüche 1 bis 14, **gekennzeichnet durch** ein Tragemittel, insbesondere einen Gürtel, einen Tragegurt, eine Tragetasche oder eine Klebverbindung, wobei das Steuergerät (6) an dem Tragemittel befestigt ist.

16. Einrichtung (9) nach einem der Ansprüche 1 bis 15, **dadurch gekennzeichnet, dass** zwischen dem Steuergerät (6) und der Ladeschale (8) induktiv über die Sendespule (12) und die Empfangsspule (15) Daten austauschbar sind.

17. Herzpumpeneinrichtung, umfassend eine Einrichtung (9) nach einem der Ansprüche 1 bis 16 und die implantierbare Herzpumpe (1).

18. System umfassend eine Einrichtung (9) nach einem der Ansprüche 1 bis 16 und ein Spiralkabel (10), wobei die Ladeschale (8) zur Versorgung der Ladeschale (8) mit elektrischer Energie mit dem Spiralkabel (10) verbunden und das Spiralkabel (10) mit der externen Energieversorgung (22) verbindbar ist.

19. System umfassend eine Einrichtung (9) nach einem der Ansprüche 1 bis 16 und die externe Energieversorgung (22), wobei die externe Energieversorgung (22) einen Wagen (36) mit einem Akkumulator (38) umfasst, wobei der Akkumulator (38) zur Versorgung der Ladeschale (8) mit elektrischer Energie mit dieser verbunden oder verbindbar ist.
